(19) Europäisches Patentamt
European Patent Office
Office européen des brevets

(11) EP 4 118 978 A1

(12) # EUROPEAN PATENT APPLICATION
published in accordance with Art. 153(4) EPC

(43) Date of publication:
**18.01.2023 Bulletin 2023/03**

(21) Application number: **20923599.3**

(22) Date of filing: **06.03.2020**

(51) International Patent Classification (IPC):
***A23L 19/00*** *(2016.01)*

(52) Cooperative Patent Classification (CPC):
**A23L 19/00**

(86) International application number:
**PCT/JP2020/009785**

(87) International publication number:
**WO 2021/176707 (10.09.2021 Gazette 2021/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Bhn Co., Ltd.**
**Tokyo 101-0054 (JP)**

(72) Inventors:
- **TANAKA, Ikuro**
  **Tokyo 101-0054 (JP)**
- **KIMURA, Masayo**
  **Tokyo 101-0054 (JP)**
- **NOZAKI, Tsutomu**
  **Tokyo 101-0054 (JP)**
- **ISHIHARA, Keno**
  **Tokyo 101-0054 (JP)**

(74) Representative: **Forrest, Stuart**
**WP Thompson**
**138 Fetter Lane**
**London EC4A 1BT (GB)**

(54) **ORAL COMPOSITION FOR INHIBITING ELASTASE AND USE THEREOF, ELASTASE INHIBITOR, AND METHOD FOR INHIBITING ELASTASE ACTIVITY BY ORAL INTAKE OF ELASTASE INHIBITOR**

(57) Provided are an oral composition for inhibiting elastase, a use of the oral composition, an elastase inhibitor, and a method for inhibiting the elastase activity by the oral intake of an elastase inhibitor, for the purpose of recovering the decrease of elastin in a biological tissue which is caused by an external factor such as ultraviolet ray, active oxygen or the like, the deterioration in a metabolic function in a living body or the like and improving an aging condition in the skin or the like. The composition for inhibiting elastase is characterized by containing a processed product of a seed of okra (Abelmoschus esculentus) or a specific compound derived from a seed of okra as an active ingredient.

EP 4 118 978 A1

## Description

FIELD OF THE INVENTION

The present invention relates to an oral composition for inhibiting elastase and use thereof, an elastase inhibitor, and a method for inhibiting the elastase activity by oral intake of the elastase inhibitor, characterized in that a processed product of okra (Abelmoschus esculentus) seeds is contained.

In particular, the present invention relates to an oral composition for inhibiting elastase, which contains cyclic peptide "OF4949-II" as an active ingredient, and use thereof, an elastase inhibitor, and a method for inhibiting the elastase activity by orally ingesting an elastase inhibitor.

2. DESCRIPTION OF THE RELATED ART

In general, biological tissues of animals and fishes are broadly classified into epithelial tissues, such as the epidermis of the skin and the surfaces of organs; muscle tissues, which mainly constitute muscles; nerve tissues, which are the nerves that stretch throughout the body, and connective tissues, which hold and support body structures. Connective tissue is also called supporting tissue, and in a broader sense, bone, cartilage, blood, fat, etc. may be classified as connective tissues.

Among these, research and development efforts have long been conducted on epithelial and supporting tissues in skin, bone, fat, etc. In recent years, for example, the generation mechanism of skin tension and elasticity loss (wrinkles, sagging, etc.) as a part of skin-aging conditions is being clarified, and elastin (elastic fibers) plays a major role in skin wrinkles and sagging.

Elastin is a type of fibrous high-molecular-weight protein found in connective tissue. It forms an extracellular matrix structure in which collagens, high-molecular-weight proteins, are lattice-like coupled and various extracellular components such as hyaluronic acid, chondroitin sulfate, and other mucopolysaccharides exist in the gaps between the collagens. This extracellular matrix structure plays a role in supporting cells and skin tissues, retaining water in intercellular spaces, maintaining skin lubricity and flexibility, and protecting skin tissues from external factors such as ultraviolet rays, dry environments, mechanical stimulation and damage, and microbial infection. These proteins and extracellular components are known to be produced by fibroblasts in vivo (Non-Patent Literature 1).

It is recognized that the elastin content in living tissues is generally about 78 - 80% in nuchae ligaments, about 50% in arteries, about 20% in lungs, and about 2 - 5% in the dermis. Another characteristic of elastin is that, unlike collagen and hyaluronic acid, it is almost 0% at human birth, increases in quantity with growth, and reaches its peak around the mid-20s. After that, it begins to decline, and after the age of 40s, it is the to be 50% or less. This suggests that elastin in living tissues may be degraded or denatured by metabolic changes associated with aging, ultraviolet rays, and other factors and that one factor may be the increased activity or excessive induction of elastase, an enzyme that degrades elastin, or the like.

From this point of view, the use of an aqueous component from okra seeds as an active ingredient to promote fibroblast proliferation has been disclosed (Patent Literature 1). In addition, various studies have been conducted and various reports and proposals have been made to prevent the reduction of the content of elastin and collagen in the dermis. As one example, retinol glycoside is disclosed as an active ingredient in cosmetics for improving skin aging (wrinkles and elasticity) (Patent Literature 2). However, retinol derivatives are known to have side effects due to excessive administration and are not safe enough.

There have also been many attempts to search for substances that inhibit the elastase activity, and so far, as plant-derived ingredients and extracts, extract of the Norwegian angelica (Patent Literature 3), extract of the Cistanche plant containing phenylethanoid glycosides (echinacosides and acteosides) (Patent Literature 4), resveratrols extracted from grape buds and vines (Patent Literature 5), pomegranate flower powder and/or extract (Patent Literature 6), extract of Ganoderma lucidum (Patent Literature 7), soy protein hydrolysate (Patent Literature 8), etc., have been proposed.

The above prior art documents describe the use of these ingredients and extracts blended in cosmetics and topical skin care products for application to the skin. However, when these ingredients and extracts are used in cosmetics and topical skin care products, there are questions and difficulties in terms of transdermal absorption of the ingredients and easy washing, etc. The preferable effects as measures against skin-aging conditions do not last, and the physiological functions of skin tissue are not essentially improved. In addition, when peptides are taken orally, there is a risk of transformation or degradation in the gastrointestinal tract, and there were only a few peptides that could express effectiveness in practical terms. Therefore, there was a need for an effective material that could improve the aforementioned skin-aging conditions.

In addition, the okra (Abelmoschus esculentus) is a plant belonging to the genus Abelmoschus Manihot and grows in tropical and temperate regions of the world and has a long history of being cultivated as a vegetable and used for food. The fruits (capsules), which contain white or yellowish-white immature seeds, are usually ingested as fresh

vegetables.

In attempts to industrially utilize extracts and components obtained by processing okra, the aforementioned Patent Literature 1, a hyaluronic acid synthesis promoting agent containing okra extract and cosmetics and food and beverages containing the agent (Patent Literature 9), and an anti-aging topical skin application containing oligopeptide derived from okra seeds and specific plant extracts (Patent Literature 10), etc. have been proposed. However, there is no reference to the relationship between okra seeds or processed products and elastin or elastase.

CITATION LIST

Non-Patent Literature

Non-Patent Literature 1: Michihiro Hattori, "Science of Skin Care," pp. 6 - 14 and 15-83, published by Shokabo, February 25, 1997.

Patent Literature

Patent Literature 1: JP-A-2018-115143
Patent Literature 2: JP-A-10-158290 (claim 1, etc.)
Patent Literature 3: JP-A-2012-201615
Patent Literature 4: JP-A-2009-263279
Patent Literature 5: JP-A-2008-239576
Patent Literature 6: JP-A-2005-53873
Patent Literature 7: JP-A-2005-23021
Patent Literature 8: JP-A-2004-182687
Patent Literature 9: JP-A-2004-51533
Patent Literature 10: JP-A-2008-74757
Patent Literature 11: JP-A-02-288890

SUMMARY OF THE INVENTION

PROBLEMS TO BE SOLVED BY THE INVENTION

In view of such current situation, the present invention is to provide an oral composition for inhibiting elastase and use thereof, an elastase inhibitor, and a method for inhibiting the elastase activity by oral intake of the elastase inhibitor, in order to restore the reduction of elastin in living tissue caused by external factors such as UV light and active oxygen and by a decrease in the metabolic function of the living body and to improve skin-aging conditions and the like.

In order to solve the aforementioned problems, the inventors have diligently studied materials that inhibit the activity of fibroblast-derived elastase and their processing method, and have found that processed okra seeds are effective.

Furthermore, as a result of continued research and development on an ingredient that inhibits the activity of fibroblast-derived elastase and its purification method, the inventors have found that "OF4949-II," a cyclic peptide obtained from fully ripe okra seeds, is an effective ingredient, resulting in the completion of the invention.

In other words, the main features of the invention are generally as follows:

(1) A composition for inhibiting elastase characterized by containing a processed product of okra (Abelmoschus esculentus) seeds.

Here, the seeds are preferably fully ripe seeds, the processed product is preferably any of an extract of okra seeds, a residue of the extraction, an enzymatically degraded product, or a sprout, the process of extraction hot water extraction, the process of enzymatic degradation is preferably hydrolysis using alkaline protease and/or neutral protease, and the elastase is preferably derived from skin fibroblasts. It is also preferable that the compositions are taken or administered orally.

(2) A composition for inhibiting elastase, characterized by containing as an active ingredient a specific compound derived from okra seeds.

Here, the seeds are preferably fully ripe seeds, and the elastase is preferably derived from skin fibroblasts. It is also preferable that the specific compound is a cyclic peptide "OF4949-II", and it is preferable that the peptide is represented by the following formula I.

[Formula I]

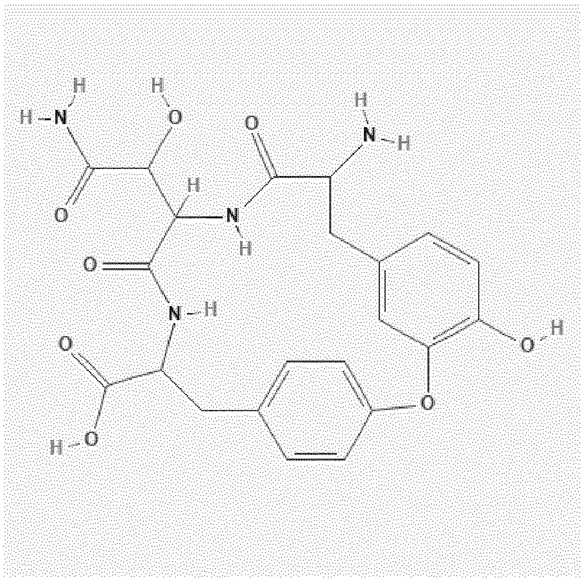

(3) A use of the composition characterized by the inhibition of the elastase activity by intake or administration of the composition.

Here, it is preferable to utilize the composition by ingesting or administering orally, and the composition for oral use is preferably the mode of a food or beverage. It is also preferable that the use of the composition is for increasing elastin on the basis of inhibition of the elastase activity in skin tissues, and moreover, it is preferable that the use of the composition is for improving an aging condition of skin tissues.

(4) A use of a cyclic peptide "OF4949-II", which is characterized by inhibiting the elastase activity.

Herein, it is preferable to utilize the composition containing the peptide by oral intake or administration, and the oral composition containing the peptide is preferably a mode of food or drink. It is also preferable that the use of the composition is for increasing elastin on the basis of inhibition of the elastase activity in skin tissues, and moreover, it is preferable that the composition is for improving an aging condition of skin tissues.

(5) An elastase inhibitor, characterized by comprising the composition for inhibiting elastase.

(6) A method for inhibiting the elastase activity characterized by oral intake of the elastase inhibitor.

EFFECTS OF THE INVENTION

The hot water extract, its residue, protein hydrolysate, and sprouts according to the present invention, which are processed products obtained from the fully ripe seeds of okra, have a strong inhibitory action on elastase derived from fibroblasts in the skin even in minute amounts, with the cyclic peptide "OF4949-II", a specific compound derived from okra seeds, as the active ingredient.

Therefore, the composition for inhibiting elastase and the elastase inhibitor of the present invention containing the peptide can promote skin turnover, improve skin problems and an aging condition (wrinkles, spots, dullness, freckles, sagging, etc.), and contribute to promoting beautiful skin. It is also expected to help maintain skin health by enhancing elastin in skin tissues and promoting regeneration of damaged skin areas, for example.

Such effects are significantly expressed by orally ingesting or administering the composition for inhibiting elastase of the present invention. Therefore, the composition for inhibiting elastase and the inhibitor comprising the composition can be effectively used for skin improvement, especially in the fields of food and beverages, pharmaceuticals, animal feed, etc., in the mode of the agent or in a form formulated in various conventional products in the aforementioned fields.

BRIEF DESCRIPTION OF THE DRAWINGS

FIG. 1 is a flowchart showing the process of obtaining the elastase inhibitory active fraction from a solution of an extract of fully ripe okra seeds.
FIG. 2 is a chromatogram of a fraction F fractionated at a concentration of 4 mg/mL.
FIG. 3 is a chromatogram of a fraction F fractionated at a concentration of 4 mg/mL.
FIG. 4 shows a chromatogram of the purified product when a fraction (5)-1 is isolated at a concentration of about 10 mg/mL.

MODE FOR CARRYING OUT THE INVENTION

The present invention is described in more detail below. First, the composition for the inhibition of elastase has the action of inhibiting elastase, an enzyme that degrades elastin present in biological tissues, particularly in the dermal tissue of the skin, and is characterized by containing processed okra seeds.

The composition for inhibiting elastase is also characterized by containing a specific compound derived from okra seeds, a cyclic peptide "OF4949-II" as the active ingredient.

Okra generally comes in pentagonal, octagonal, round, and other varieties of capsules, and in green, purple-red, white, and other colors. The okra of the present invention is not limited to these varieties, and any type of okra can be used.

In the present invention, it is preferable to use fully ripened seeds of the aforementioned okra as raw material. The fully ripe seeds refer to seeds harvested from the fruits (capsules) from which the plant body has grown or seeds which the plant body has spontaneously dispersed, dark green to blackish brown in color, around 5 mm in diameter, and have the ability to germinate if sown.

Immature seeds, on the other hand, are contained within the developing capsules, are white to yellowish-white in color, approximately 2 - 4 mm in diameter, and are usually served with the capsules for food. Fully matured okra seeds are commercially available from seed companies for growing okra, and are easy to use.

The aforementioned okra seeds are preferably collected as the processed products described below, i.e., okra seed extract, the extract residue, enzymatic hydrolysis products or sprouts.

Extracts of okra seeds can be produced, for example, by the following processing steps. After the hulled or unpeeled seeds are coarsely or finely crushed and water is added to make a dispersed solution, extraction is performed at room temperature or under heat, while standing still or stirring as appropriate. The extract is then treated by centrifugation, filtration, or the other conventional method to remove insoluble matter, and preferably concentrated at room temperature or lower, and dried or freeze-dried to produce the extract of the present invention.

In the aforementioned extraction process, when hot water extracts are produced, the aqueous dispersion is set at about 30 to about 90°C, more preferably about 40 to about 60°C, and is extracted for about 10 minutes to several days, more preferably for about 30 minutes to several hours, and when low-temperature extracts are produced, the temperature is set at about 10 to about 30°C, more preferably about 15 to about 25°C, and is soaked for about 30 minutes to several days, more preferably for several hours to one day.

The extraction residue of okra seeds can be produced by concentration drying or freeze-drying the insoluble material removed in the extraction process described above using a conventional method. In this case, the degree of drying is preferably such that the moisture content is reduced to 5% or less by mass in order to prevent contamination by molds and the like. In relation to the desired effect of the invention, it is preferable that the aforementioned residue of the present invention be the residue that is separated when producing the aforementioned low-temperature extract, the low-temperature extraction residue.

An enzymatically degraded product of okra seeds can be produced by the following processing treatment. Okra seeds are triturated to make a water dispersion, to which is added about 0.1 to about 5% by mass of protein hydrolyzing enzyme per seed, preferably about 0.5 to about 2% by mass of protein hydrolyzing enzyme per seed, and the hydrolysis process is carried out under normal or elevated temperature (about 30 to about 90°C, more preferably about 40 to about 60°C) for about 10 minutes to several days, more preferably 30 minutes to several hours, by standing or stirring as needed. After the treatment, the enzyme is inactivated by heat according to the usual method, and then the insoluble

matter is separated by centrifugation or filtration, and preferably concentrated, dried, or freeze-dried at room temperature or lower temperature. This can result in the enzymatically degraded product of okra seeds. In the present invention, instead of the aforementioned water dispersion, the extracted product or extraction residue of okra seeds obtained by the aforementioned process may be dissolved or dispersed in water and enzymatically treated in the same manner.

Although any kind or type of protein hydrolyzing enzymes can be used, alkaline proteases and/or neutral proteases are preferred in the present invention, and alkaline proteases are even more preferable. Here, alkaline proteases can be used as long as they show action at a substrate pH of approximately 6.5 to 12, and neutral proteases are acceptable if they act at a pH of approximately 5 - 8 of the substrate. Each protease may be used in combination with not only one but also two or more proteases, and it is convenient to use commercial products such as those described below.

The following specific examples can be given as alkaline proteases. However, the present invention is not limited by these in any way.

"Protin SD-AY10", "Protease P "Amano" 3SD", "Proleather (registered trademark) FG-F" (all by Amano Enzyme Inc.), "Alcalase (registered trademark) 2.4LFG" (Novozymes A/S), "Orientase (registered trademark) 22BF" (HBI Enzyme Inc.), "Aroase (registered trademark) XA-10" (Yakult Pharmaceutical Industry Co., Ltd.), "Sumizyme (registered trademark) MP" (SNBL, Ltd.), "Bioprase OP, SP-20FG, AL-15FG, 30G, APL-30 and 30L" (Nagase ChemteX Corporation), "OPTIMASE (registered trademark) PR89L", "MALTIFE CT (registered trademark) PR6L" (all by Danisco US, Inc.), "Proteinase K" and "Chymotrypsin" (all by F. Hoffmann-La Roche AG), etc.

The following neutral proteases can be exemplified, but the present invention is not limited by them in any way.

"Flavourzyme (registered trademark)", "PROTAMEX (registered trademark) MG", "Neutrase" (all by Novozymes A/S), "Pancidase (registered trademark) P and MP", "Aroase (registered trademark) AP-10, NP-10 and NS" (all by Yakult Pharmaceutical Industry Co. Ltd.), "Nucleisin (registered trademark)", "Orientase (registered trademark) 10NL and 90N" (all by HBI Enzyme Inc.), "Protin P" (Daiwa Fine Chemicals Co., Ltd.), "Protease A "Amano" G", "Papain W40", "Bromelain F" (all by Amano Enzyme Co., Ltd.), "Sumizyme (registered trademark) LP and LPL" (SNBL, Ltd.), "Food Purified papain", "Denazyme AP" (all by Nagase ChemteX Corporation), "Trypsin" (F. Hoffmann-La Roche AG), etc.

Okra seed sprouts can be produced by the known method, i.e., germinating the seeds, growing them into a "kaiware" (i.e., white radish sprouts)-type plant, harvesting them after growing until green leaves appear and the stem length is about 5 to 15 cm, washing them in water, and then drying or freeze-drying them.

The aforementioned processed product obtained by processing as described above is a highly complex composition containing various components such as proteins, polysaccharide-protein complexes, polysaccharides, etc., contained in okra seeds and/or bonds between their hydrolyzed peptides, amino acids, oligosaccharides, monosaccharides or oligosaccharides, and peptides or amino acids.

As a result of their intensive research on the complex composition of the aforementioned processed okra seed product, the inventors have discovered that the cyclic peptide "OF4949-II" is contained as the active ingredient. The cyclic peptide "OF4949-II" is known to be a potent inhibitor of the action of aminopeptidase B and to be produced by microorganisms belonging to the genus Penicillium (Patent Literature 11), but there are no reports of its presence in the fully ripe okra seeds.

The cyclic peptide "OF4949-II" can be produced, for example, by the following purification process: Extraction of the fully ripe okra seeds is carried out under heat while keeping the seeds still or stirring them as needed. The extracted solution is then treated by centrifugation, filtration, or other conventional methods to remove insoluble matter, and preferably concentrated and dried or freeze-dried at room temperature or lower, to obtain the extracted product. The extracted product is then subjected to flash chromatography or high-performance liquid chromatography to concentrate the desired fraction and repeat the purification process.

In the aforementioned purification process, the cyclic peptide "OF4949-II" is finally identified by mass spectrometry or nuclear magnetic resonance spectrometry to confirm its identity.

In the present invention, not only the cyclic peptide "OF4949-II" derived from okra seeds, but also the cyclic peptide "OF4949-II" obtained from microorganisms as described above can be used as the active ingredient having elastase inhibitory action in the present application, and the same effect can be expected.

In the present invention, the aforementioned processed okra seed product can be used as is or in combination with known excipients such as dextrin, cellulose, gelatin, purified water, etc. to prepare the liquid, powder, granule, or capsule inhibitor containing the composition for inhibiting elastase of the present invention. The content of processed okra seed product in these agents is generally 20 mass percent or more, and if the content is less than 20 mass percent, the desired effect of the invention will be difficult to manifest, so it is preferable that the processed okra seed product/excipient (mass ratio) is preferably 100 to about 20/0 to about 80, and it is even more preferable that it be 100 to about 50/0 to about 50.

The composition for inhibiting elastase of the present invention and the elastase inhibitor containing the composition can be used as a cosmetic means to improve the aforementioned problems of the skin, restore an aging condition, and promote beautiful skin since oral intake or administration of the composition inhibits the elastase activity in the skin

tissue and prevents elastin reduction.

In this preferred embodiment, the elastase inhibitor of the present invention can be shaped into solid (powder or granules), gel (jelly), paste, or liquid (beverage or drink) products such as food products, pharmaceuticals, and animal feed. In addition, the aforementioned various products can be made by the usual method by using known additives (surfactants, thickeners, antioxidants, colorants, fragrances, etc.) used to manufacture these products and known materials that do not contradict the purpose of the invention (such as materials derived from plants and animals with known effects such as promoting fibroblast proliferation, promoting the production of extracellular components such as collagen and hyaluronic acid, preventing skin aging, and promoting beautiful skin, etc.) in combination as appropriate.

[Examples]

The invention is described in detail below with examples, but the invention is not limited thereby. In the following, unless otherwise noted, parts and percentages are on a mass basis.

[Example 1]

[Production examples of the processed okra seed product]

Production Example 1

Ripe okra seeds produced in Ibusuki, Kagoshima Prefecture, were coarsely ground in a mill, and a water dispersion (pH 7.5) was prepared by adding 300 mL of distilled water to 100 g of the seeds and heating to 58°C with stirring. For this water dispersion, one part of the proteolytic enzyme (product name: "Orientase 22BF", alkaline protease, manufactured by HBI Enzyme Inc.) was added while gentle stirring was continued for 5 hours. After this time, the enzyme was inactivated (at 80°C for 30 min), and the insoluble material was removed by centrifugation, followed by lyophilization, resulting in the enzymatically degraded product (Sample 1).

Comparative Sample 1

As a comparative sample 1, a commercial product derived from lychee seeds (trade name: "Lychee Seed Extract-WSP" manufactured by Oryza Oil & Fat Chemical Co., Ltd.), a material known to have elastase inhibitory action, was used.

Comparative Sample 2

As a comparative sample 2, a commercial product of resveratrol-containing composition (product name: "Resveratrol ε" manufactured by BHN Co., Ltd.), a material known to have elastase inhibitory action, was used.

Comparative sample 3

As a comparative sample 3, a commercial product of Peucedanum japonicum (Botan Boufuu) leaf powder (product name: "Botan Boufuu Leaf Powder", manufactured by BHN Co., Ltd.), a known plant-derived material, was used.

Comparative Sample 4

As a comparative sample 4, a commercial product of sesame leaf powder (product name: "sesame leaf powder" manufactured by BHN Corporation), a known plant-derived material, was used.

Comparative sample 5

As a comparative sample 5, a commercial product of okra seed powder (seller: Matsumoto Trading Co., Ltd., trade name: "Myoxinol LS 9736"), a known okra-derived material, was used.

Production Example 2

The fully ripe seeds used in Production Example 1 were coarsely crushed, 100 g were added to 800 mL of water, heated at 80 - 90°C for 60 minutes, and were then cooled to room temperature, and subjected to precise filtration (filter paper) to collect the filtrate. To the filtrate residue, 600 mL of water was added again, and the filtrate was collected

by processing in the same manner. Both the filtrates were combined and concentrated under reduced pressure, then lyophilized and ground to produce the extracted product (Sample 2).

Production Example 3

The fully ripe seeds used in Production Example 1 were coarsely crushed, 100 g were added to 800 mL of water, soaked overnight at 15 - 25°C, and were then subjected to precise filtration (filter paper) to collect the filtrate, and the filtrate was concentrated under reduced pressure, freeze-dried and crushed to produce the extracted product (Sample 3).

Production Example 4

The residue after the filtration described in Production Example 3 was lyophilized and crushed to produce the extraction residue (Sample 4).

Production Example 5

The same fully ripe seeds as in Production Example 1 were soaked in water at 0°C to room temperature for 8 to 20 hours to remove the water, then sown in growing containers, stored in a dark room at room temperature to 30°C and 75 to 90% humidity, and germinated by spraying water as needed. After the germination, they were moved to a greenhouse and grown under sunlight at a room temperature of 25 - 30°C, with water sprayed as needed. Kaiware-type sprouts were obtained after 5 to 7 days. The sprouts were rinsed, freeze-dried, and crushed to produce the crushed sprout product (Sample 5).

Comparative Production Example 1

In Production Example 1, the enzymatically degraded product (Comparative Sample 6) was obtained by the same process except that the fully ripe seeds were substituted for unripe seeds.

Comparative Production Example 2

The enzymatically degraded product (Comparative Sample 7) was obtained by the same process as in Production Example 1, except that the fully ripe seeds were replaced with a commercial product (product name: "Okra Powder" manufactured by Yell Co., Ltd.) derived from edible parts of okra (the capsules containing the immature seeds. The same applies hereinafter.).

Comparative Production Example 3

The extracted product (Comparative Sample 8) was obtained by the same process as in Production Example 2, except that the fully ripe seeds were replaced with a commercial product (product name: "Okra Powder" manufactured by Yell Co., Ltd.) derived from edible parts of okra.

Test Examples

The effects of each of the aforementioned samples on the elastase activity were examined by methods described below.

Test Example 1: Elastase Inhibiting Action (Part 1)

Sample 1 and comparative samples 1 - 3 described above were used as test samples for measuring the elastase activity. Each test sample was dissolved in 100 mM Tris-hydrochloric acid buffer (pH 8.0) to prepare a test solution having a final concentration of 40.0 to 1000.0 μg/mL. At this time, if it was difficult to dissolve, DMSO was added within 10%.

In 96 microplates, 25 μl of this test solution was mixed with 25 μl of human skin fibroblast-derived elastase solution (Here, the elastase solution is prepared by pre-culturing human skin fibroblasts (manufactured by Kurabo Industries Ltd.) so as to be confluent, culturing in DMEM medium containing 2% FBS for 24 hours, and then washing twice in PBS (-), followed by adding 0.1% triton X-100 and dissolving by sonication. This lysate was centrifuged at 12000 rpm for 5 minutes, the supernatant was collected, and the lyophilized powder was used as a crude enzyme powder, which was dissolved in a buffer solution in each test.) and prewarmed at 37°C for 5 minutes.

As a control, a mixture of the elastase solution with 100 mM Tris-HCl buffer and DMSO alone added thereto was also treated in the same way.

Next, a reaction was initiated by adding 50 μl of a solution containing N-succinyl-trialanine-p-nitroanilide as substrate (the solution produced by dissolving the substrate in a minimal volume of DMSO and adding 100 mM Tris-HCl buffer (pH 8.0) to make 5 mM). After 2 hours, nitroaniline, which was released by the elastase activity, was measured as 405 nm wavelength light using a spectrophotometer (Microplate Reader SH9000 Lab, Corona Electric Co., Ltd.), and the elastase inhibition rate was calculated from the absorbance of the specimen relative to the absorbance of the control to which no test sample was added. The elastase inhibition rate was calculated from the following formula:

$$\text{Elastase inhibition rate (\%)} = \{1 - (\text{control} - \text{sample}) / (\text{control} - \text{blank})\} \times 100$$

The results were shown in Table 1. In the same table, the degree of elastase inhibiting action derived from the fibroblasts was expressed as a relative value when the value of the control test conducted at the same time was 0. The data in the same table showed that the enzymatic treatment of the fully ripe seeds (sample 1) exhibited a remarkably high elastase inhibiting action at all concentrations of the test samples. In contrast, the lychee seed extract (Comparative Sample 1) and the resveratrol-containing composition (Comparative Sample 2), which were already known to inhibit elastase, showed an increased inhibitory action at high concentrations but a small inhibitory action at low concentrations, and in addition, it was confirmed that the plant-derived button flower leaf powder (Comparative Sample 3) and the sesame leaf powder (comparative sample 4) had a weak inhibitory action at any concentrations.

Table 1 Degree of elastase inhibiting action

| Test sample and concentration (μg/mL) | | Elastase inhibiting action (Inhibition rate) |
|---|---|---|
| Control | | 0.00 |
| Sample 1 | 40 | 98.11 |
| | 200 | 99.56 |
| | 1000 | 99.85 |
| Comparative Sample 1 | 40 | 2.55 |
| | 200 | 32.20 |
| | 1000 | 88.35 |
| Comparative Sample 2 | 40 | 9.15 |
| | 200 | 39.66 |
| | 1000 | 90.85 |
| Comparative Sample 3 | 40 | 1.50 |
| | 200 | 4.02 |
| | 1000 | 18.68 |
| Comparative Sample 4 | 40 | 9.20 |
| | 200 | 15.54 |
| | 1000 | 32.69 |

Test Example 2: Elastase Inhibiting Action (Part 2)

In this test example, the effects of processed products other than the enzymatically degraded product of okra seeds and processed products other than the fully ripe seeds on elastase inhibiting action were investigated. Here, the measurement of elastase inhibitory action and the evaluation of the degree of inhibition were conducted in the same manner as in Test Example 1, but the final concentrations of the test solutions ranged from 0.4 to 10.0 μg/mL. The test samples used were the aforementioned sample 1, samples 2 - 5, and comparative samples 5 - 8.

The results were shown in Table 2. In the same table, the degree of elastase inhibiting action (% inhibition) was expressed as a relative value when the value of the control test conducted at the same time was 0.

The data in the same table confirmed that the extracted products (samples 2 and 3), the extraction residue (sample 4), and the sprout (sample 5), which were the processed products of the fully ripe okra seeds, exhibited the elastase inhibitory action as strong as that of the enzyme degraded product (sample 1), while it was confirmed that the elastase inhibitory action was inferior in the case of the okra seed-derived powder (Comparative Sample 5), and that

little elastase inhibitory action was observed when the immature seeds were enzymatically degraded (Comparative Sample 6), when the edible part (the capsule including the immature seeds) was enzymatically treated (Comparative Sample 7) and when the hot water extraction was used (Comparative Sample 8).

Table 2 Degree of elastase inhibiting action

| Test sample and concentration (μg/mL) | | Elastase inhibiting action (Inhibition rate) |
|---|---|---|
| Control | | 0.00 |
| Sample 1 | 0.4<br>2.0<br>10.0 | 22.31<br>57.56<br>87.08 |
| Sample 2 | 4<br>2.0<br>10.0 | 14.59<br>42.19<br>79.60 |
| Sample 3 | 0.4<br>2.0<br>10.0 | 15.65<br>39.61<br>73.26 |
| Sample 4 | 4<br>2.0<br>10.0 | 16.00<br>42.08<br>76.60 |
| Sample 5 | 0.4<br>2.0<br>10.0 | 19.65<br>65.75<br>86.86 |
| Comparative Sample 5 | 0.4<br>2.0<br>10.0 | 4.18<br>14.20<br>29.74 |
| Comparative Sample 6 | 0.4<br>2.0 | 3.81<br>4.68 |
| | 10.0 | 4.45 |
| Comparative Sample 7 | 0.4<br>2.0<br>10.0 | 4.23<br>4.00<br>6.32 |
| Comparative Sample 8 | 0.4<br>2.0<br>10.0 | 3.56<br>4.41<br>7.95 |

Test Example 3: Human Monitoring Test

Forty adult female volunteers (30 - 55 years old, mean age: 44.5) who agreed to participate in the following study were divided into two groups of 20 subjects. One group was given sample 1 (100 mg) and the other group was given a placebo (100 mg of dextrin), taken orally twice a day for four weeks. The skin changes before and after the intake were evaluated using a questionnaire survey to determine whether there was any improvement in such items as skin dryness, spots, wrinkles, elasticity, dullness, sagging, laugh lines, makeup application, pore sizes, etc.

The results showed that in the sample 1 group, 17 people's skin improved, 2 people's skin did not change, and 1 person's skin worsened compared to before the intake, while in the placebo group, 2 people's skin improved, 14 people's skin did not change, and 4 people's skin changed for the worse. The 17 people who had skin changes had two or more choices of improvement, especially in the items of wrinkles, elasticity, sagging, and laugh lines. They also selected improvements in skin dryness and pore sizes, which were not seen in the placebo group.

The results suggested that the processed okra fully ripe seed products were useful for skin improvement by inhibiting the elastase activity.

[Example 2]

[Example of production of the cyclic peptide "OF4949-II"]

To 5 g of the extracted product obtained from the fully ripe okra seeds, 10 mL of distilled water was added, stirred well, sonicated, centrifuged, or allowed to stand, and the supernatant was collected and used as a fractionated sample. One mL of this fractionated sample was placed in a chromatograph tube packed with TOYOPEARL HW-40F (Tosoh Corporation) preparative packing material, and a constant volume of 20% methanol has flowed through the tube.
The sequentially eluting solutions were fractionated every 3 mL into glass test tubes as shown in the flow chart in FIG. 1.

Test Example

The effects of each of the above fractions on the elastase activity were examined by the method described below.

Test example: Elastase inhibiting action

Each of the above fractions was used as test sample for measuring the elastase activity.
The test method was carried out in the same manner as in Test Example 2 above, with the final concentration of the test solution ranging from 0.4 to 10.0 μg/mL.
The results were shown in Table 3. In the same table, the degree of elastase inhibiting action of each fraction was expressed as a relative value when the value of the control test conducted at the same time was 0.
The data in the same table showed that the fraction F exhibited a remarkably high elastase inhibiting action (%) at all concentrations of the other fractions.

Table 3 Degree of elastase inhibiting action in fractions A - F

| | 0.4 μg/mL | 2.0 μg/mL | 10.0 μg/mL |
|---|---|---|---|
| Fraction A | 6.7 | 2.7 | 0.8 |
| Fraction B | 8.8 | 5.9 | 12.5 |
| Fraction C | 8.3 | 7.3 | 10.4 |
| Fraction D | 7.8 | 3.8 | 6.7 |
| Fraction D | 6.0 | 13.4 | 5.4 |
| Fraction F | 16.8 | 40.1 | 74.9 |

The aforementioned fraction F was then purified by the following process. The aforementioned fraction F was concentrated, dried, and dissolved in distilled water so as to have a concentration of about 1 - 5 mg/mL. After the dissolution, gradient analysis was performed using 5C18-MS-II (Nacalai Tesque, Inc.), which was a C18 column, at a flow rate of 0.7 mL/min using high-performance liquid chromatography (HPLC), and 5 types of fractions ((1) - (5) in order of elution) were obtained, and the elastase inhibitory activity of each fraction was measured in the same manner. As a result, the highest inhibitory activity was identified in the fraction (5).
In the gradient analysis, water containing 0.1% formic acid (mobile phase A) and acetonitrile (mobile phase B) were used at a gradient as mobile phases in the HPLC, and the proportion of the mobile phase B was 0 - 5 (min): 0%, 15 (min): 50%, and 15 - 25 (min): 80%.
Furthermore, when the fraction (5) was divided into three fractions ((5)-1 to 3) and fractionated under the same conditions, the high inhibitory activity was confirmed practically only in (5)-1. The results were shown in Table 4.
Chromatograms of the fraction F fractionated at a concentration of 4 mg/mL were shown in Fig. 2 and Fig. 3. In FIG.s 2 and 3, the vertical axis represented UV absorption intensity (mV/cm) and the horizontal axis represented retention time (min/cm).

Table 4 Degree of elastase inhibitory action in fractions (5)-(5)-1-3

| | 0.4 μg/mL | 2.0 μg/mL | 10.0 μg/mL |
|---|---|---|---|
| Fraction (5) | 85.55 | 93.55 | 95.18 |
| Fraction (5)-1 | 81.60 | 92.78 | 95.53 |

(continued)

| | 0.4 μg/mL | 2.0 μg/mL | 10.0 μg/mL |
|---|---|---|---|
| Fraction (5)-2 | 4.56 | 16.60 | 47.38 |
| Fraction (5)-3 | 2.41 | 5.76 | 19.86 |

The fraction (5)-1, which had the high inhibitory action, was repeated in the same manner to obtain a sufficient amount, and then the gradient analysis was performed using the amino column Intrada Amino Acid (Imtakt Corporation) at a flow rate of 0.6 mL/min using the HPLC. The peak at about 10.5 minutes was collected and purified.

In the gradient analysis, water containing 0.1% formic acid (mobile phase A) and acetonitrile (mobile phase B) were used at a gradient as the mobile phases in the HPLC, and the proportion of the mobile phase B was 0 - 3 (min): 90%, 10 (min): 0%, 10 - 15 (min): 0%.

For example, a chromatogram of the purified product when the fraction (5)-1 was isolated at a concentration of about 10 mg/mL was shown in FIG. 4. In FIG. 4, the vertical axis was the UV absorption intensity (mV) and the horizontal axis was the retention time (min).

The purified product obtained by the aforementioned process was subjected to direct introduction/mass spectrometry (DI/MS) and nuclear magnetic resonance analysis (NMR) to estimate its molecular weight: 472, molecular formula: $C_{22}H_{24}N_4O_8$, and it was confirmed to be the "OF4949-II" (CAS number: 93375-50-9), the cyclic peptide shown in Formula I above.

Prototype Example 1: Hard capsule formulation

The aforementioned sample 1 was placed in a capsule filling machine and the gelatin-coated hard capsule formulation with an internal volume of 100 mg per capsule was prototyped according to the usual method. A hard capsule formulation was made on a trial basis. The hard capsule formulation (containing the fully ripe okra seeds of Production Example 1) was orally ingested in a monitoring study and was found to be useful for improving skin. Therefore, this tablet can be used as an orally ingestible dietary supplement, drug, or animal feed for skin improvement.

Prototype Example 2: Tablets

Tablets were prototyped by tableting the following raw materials in the usual way. One of the above-mentioned samples 1 to 5 was used as fully ripe okra seeds. All of these tablets were stable and easy to take, and can be used as nutritional supplements or medicines.

| (Ingredients) | (Mass (mg) per tablet) |
|---|---|
| 1. fully ripe okra seeds | 100 |
| 2. dextrin | 100 |
| 3. potato starch | 49 |
| 4. microcrystalline cellulose | 20 |
| 5. synthetic aluminum silicate | 30 |
| 6. calcium stearate | 1 |

Prototype Example 3: Nutritional Supplement

The above sample 1 was 100 mg, leucine 400 mg, isoleucine 250 mg, valine 200 mg, and arginine 300 mg, and these mixed and sifted powders were packaged and filled to make a prototype dietary supplement. This dietary supplement can be used not only for humans but also for pet food.

Prototype Example 4: Food (Noodles)

A prototype soba noodle was made by mixing 10 g of the aforementioned sample 1, 350 g of buckwheat flour, and 150 g of strong flour (enriched flour), mixing them together, passing them through a sieve, and mixing 150 ml of water. This was compatible with the commercial product in flavor and texture.

Prototype Example 5: Soft drink and nutritious supplement drink

To 100 mL of commercially available soft drink and nutritious supplement drink, 500 mg or 100 mg of Sample 1 or Sample 2 described above was added and thoroughly mixed to make a prototype beverage. The beverage was comparable with the original soft drink and nutritious supplement drink in flavor and shelf stability. It can be used as a soft drink or a nutritious supplement drink.

[Industrial Availability]

The processed products obtained from the fully ripe seeds of okra, especially the cyclic peptide "OF4949-II" obtained from the fully ripe seeds of okra, has an inhibitory action on the elastase activity derived from fibroblasts, so that oral intake or administration of the product can restore the original physiological function of skin and can be effectively used as a food, medicine, animal feed, etc. that are useful for improving skin problems, promoting beautiful skin, and early recovery from skin damage.

## Claims

1. A composition for inhibiting an elastase, **characterized by** containing a processed product of a seed of okra (Abelmoschus esculentus) or a specific compound derived from the seed of okra as an active ingredient.

2. The composition according to claim 1, wherein the seed is a fully ripe seed.

3. The composition according to claim 1 or 2, wherein the processed product is an extracted product, an extraction residue, an enzymatically degraded product, or a sprout of the seed of okra.

4. The composition according to claim 3, wherein the extracted product is a hot water extracted product.

5. The composition according to claim 3 or 4, wherein the enzymatically degraded product is a product hydrolyzed by an alkaline protease and/or a neutral protease.

6. The composition according to any one of claims 1 to 5, wherein the elastase is derived from a skin fibroblast.

7. The composition according to any one of claims 1 to 6, wherein the specific compound is a cyclic peptide "OF4949-II".

[Chemical formula 1]

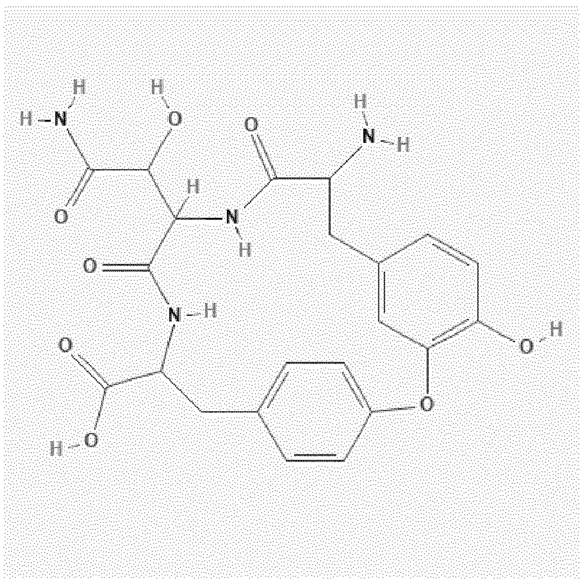

**8.** Use of the composition according to any one of claims 1 to 7, **characterized by** inhibiting elastase activity by intake or administration of the composition.

**9.** An elastase inhibitor comprising the composition according to any one of claims 1 to 7.

**10.** A method for inhibiting elastase activity, **characterized by** orally ingesting the elastase inhibitor according to claim 9.

**11.** Use of a cyclic peptide "OF4949-II", **characterized by** inhibiting elastase activity.

**12.** The use of the cyclic peptide "OF4949-II" according to claim 11 for an elastin increase on basis of an elastase inhibiting action in skin tissue.

**13.** The use of the cyclic peptide "OF4949-II" according to claim 11 for improving an aging condition of skin tissue.

## FIG. 1

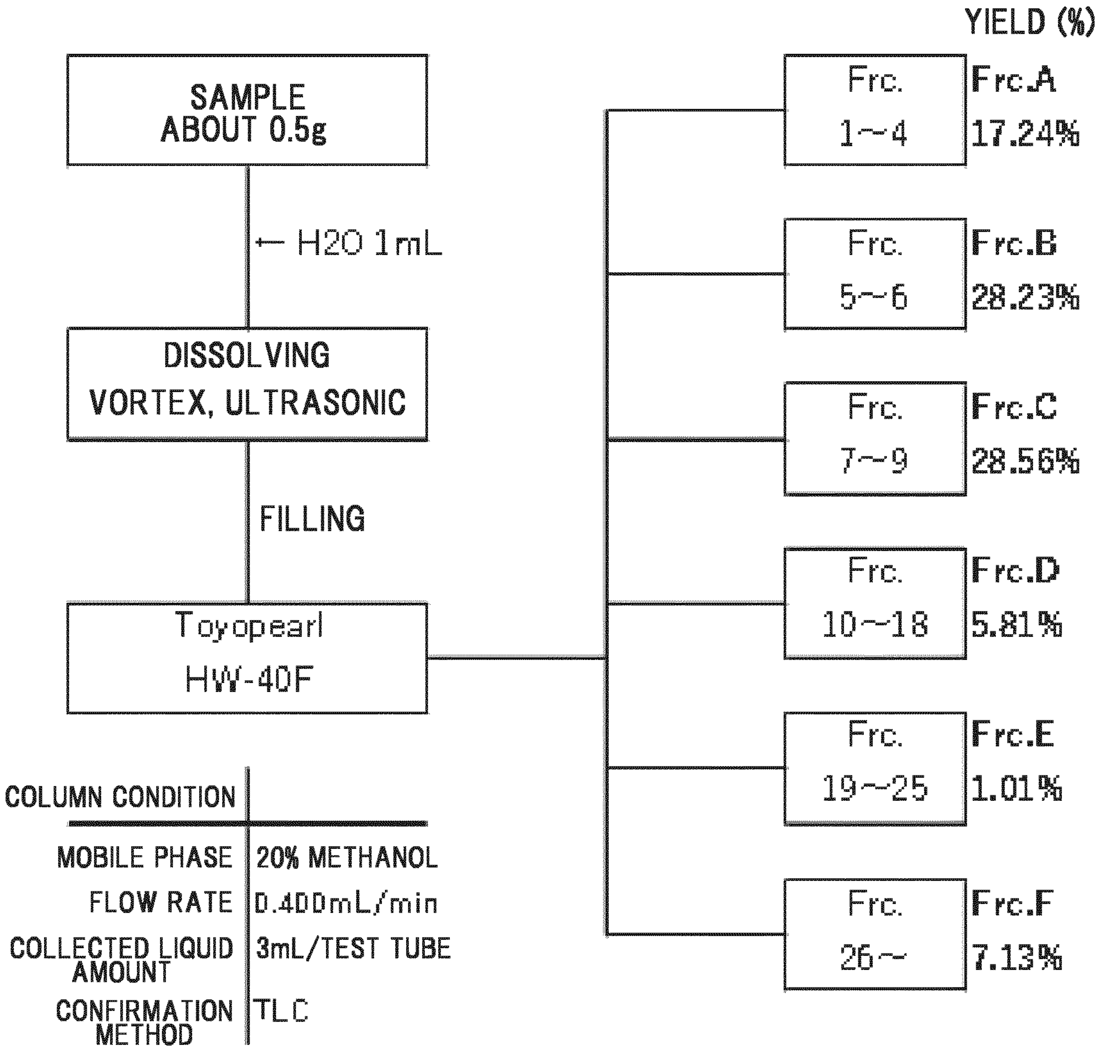
YIELD (%)
SAMPLE
ABOUT 0.5g
← H2O 1mL
DISSOLVING
VORTEX, ULTRASONIC
FILLING
Toyopearl
HW-40F
COLUMN CONDITION
MOBILE PHASE
20% METHANOL
FLOW RATE
0.400mL/min
COLLECTED LIQUID AMOUNT
3mL/TEST TUBE
CONFIRMATION METHOD
TLC
Frc.
1~4
Frc.A
17.24%
Frc.
5~6
Frc.B
28.23%
Frc.
7~9
Frc.C
28.56%
Frc.
10~18
Frc.D
5.81%
Frc.
19~25
Frc.E
1.01%
Frc.
26~
Frc.F
7.13%

## *FIG. 2*

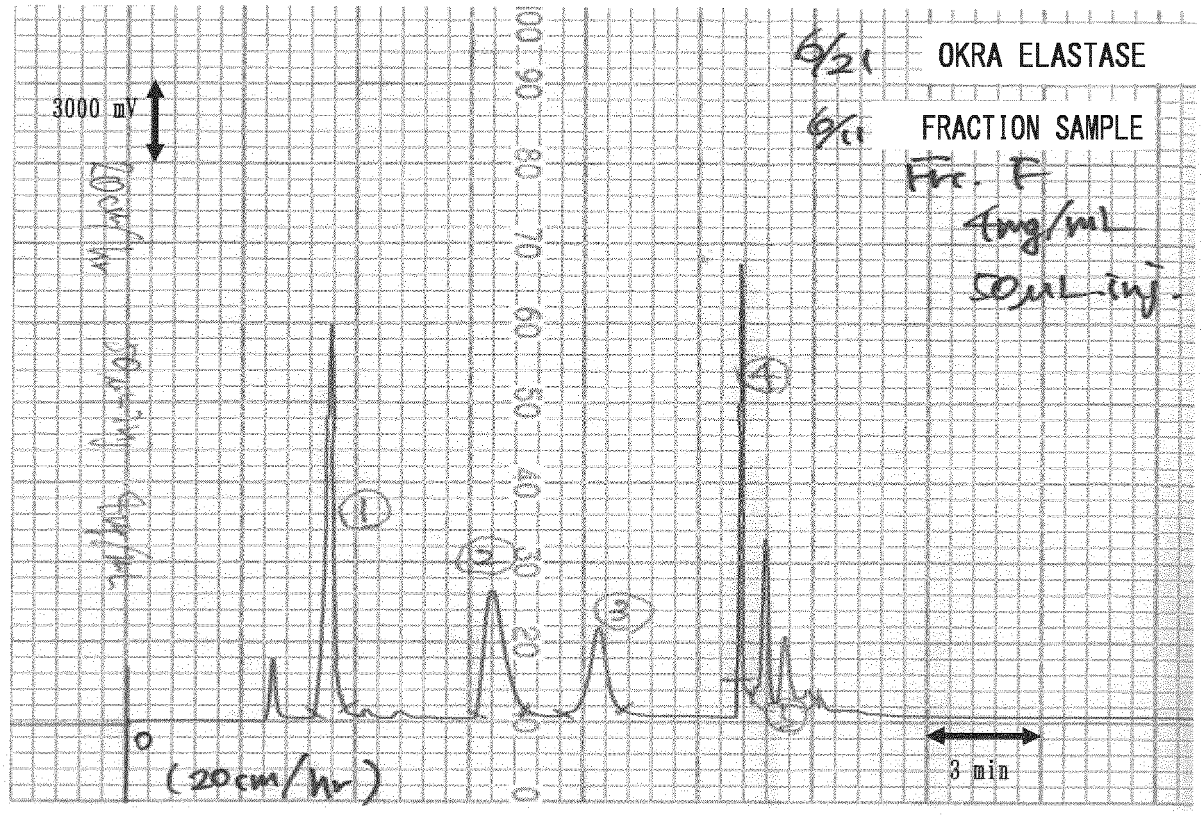
OKRA ELASTASE
FRACTION SAMPLE
3000 mV
3 min
(20cm/hr)
Frc. F
4mg/mL
50μL inj.

## *FIG. 3*

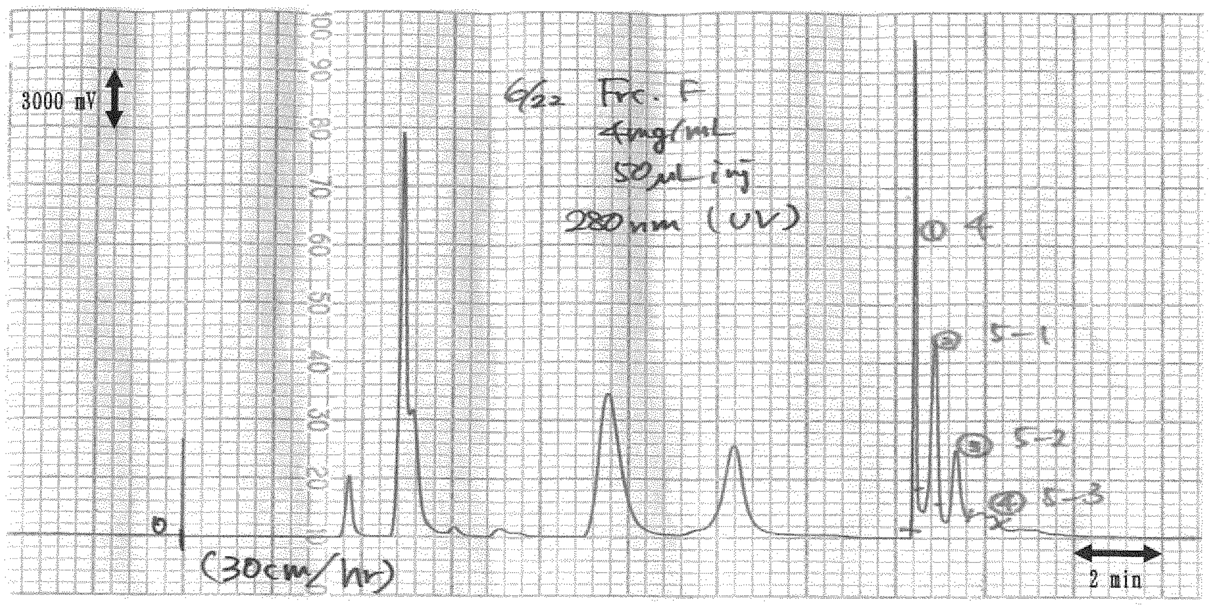
3000 mV
2 min
(30cm/hr)
Frc. F
4mg/mL
50μL inj
280nm (UV)
5-1
5-2
5-3

*FIG.* 4

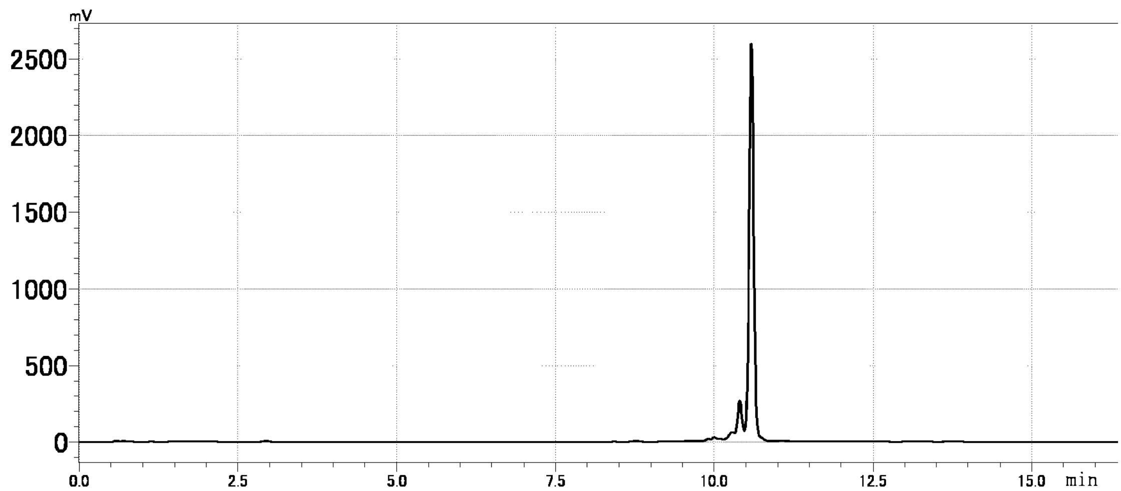

**INTERNATIONAL SEARCH REPORT**

International application No. PCT/JP2020/009785

A. CLASSIFICATION OF SUBJECT MATTER
Int. Cl. A23L19/00(2016.01)i
FI: A23L19/00 Z

According to International Patent Classification (IPC) or to both national classification and IPC

B. FIELDS SEARCHED

Minimum documentation searched (classification system followed by classification symbols)
Int. Cl. A23L19/00

Documentation searched other than minimum documentation to the extent that such documents are included in the fields searched
Published examined utility model applications of Japan 1922-1996
Published unexamined utility model applications of Japan 1971-2020
Registered utility model specifications of Japan 1996-2020
Published registered utility model applications of Japan 1994-2020

Electronic data base consulted during the international search (name of data base and, where practicable, search terms used)
JSTPlus/JMEDPlus/JST7580 (JDreamIII), CAplus/MEDLINE/EMBASE/BIOSIS/WPIDS (STN), FSTA (STN)

C. DOCUMENTS CONSIDERED TO BE RELEVANT

| Category* | Citation of document, with indication, where appropriate, of the relevant passages | Relevant to claim No. |
|---|---|---|
| Y<br>A | ARAPITSAS, Panagiotis, Identification and quantification of polyphenolic compounds from okra seeds and skins, Food Chemistry, 2008, vol. 110, pp. 1041-1045, summary, fig. 2, 3, table 1, summary, fig. 2, 3, table 1 | 1-5, 8-10<br>6-7, 11-13 |
| Y<br>A | GARINI, Marina et al., Procyanidins from Vitis vinifera seeds inhibit the respiratory burst of activated human neutrophils and lysosomal enzyme release, Planta Med., 2001, vol. 67, pp. 714-717, summary, fig. 2, summary, fig. 2 | 1-5, 8-10<br>6-7, 11-13 |
| A | JP 2018-115143 A (BHN KK) 26 July 2018, claims 1-7, paragraphs [0001], [0016] | 1-13 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22.05.2020 | 02.06.2020 |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No. PCT/JP2020/009785

C (Continuation). DOCUMENTS CONSIDERED TO BE RELEVANT

| Category* | Citation of document, with indication, where appropriate, of the relevant passages | Relevant to claim No. |
|---|---|---|
| A | SANO, Susumu et al., OF4949, NEW INHIBITORS OF AMINOPEPTIDASE B IV. EFFECTS OF OF4949 AND ITS DERIVATIVES ON ENZYME SYSTEMS, THE JOURNAL OF ANTIBIOTICS, 1987, vol. XL, no. 4, pp. 512-518, summary, table 2 | 1-13 |
| A | HOMSY, Rania et al., Characterization of Human Skin Fibroblasts Elastase Activity, J. Invest. Dermatol., November 1988, vol. 91, no. 5, pp. 472-477, table 1 | 1-13 |
| A | UMEZAWA, Hamao et al., BESTATIN, AN INHIBITOR OF AMINOPEPTIDASE B, PRODUCED BY ACTINOMYCETES, THE JOURNAL OF ANTIBIOTICS, January 1976, vol. XXIX, no. 1, pp. 97-99, p. 99, left column, paragraph [0002] | 1-13 |
| A | TSUKAHARA, Kazue et al., Selective Inhibition of Skin Fibroblast Elastase Elicits a Concentration-Dependent Prevention of Ultraviolet B-Induced Wrinkle Formation, THE JOURNAL OF INVESTIGATIVE DERMATOLOGY, 2001, vol. 117, no. 3, pp. 671-677, summary | 1-13 |

Form PCT/ISA/210 (continuation of second sheet) (January 2015)

**INTERNATIONAL SEARCH REPORT**
Information on patent family members

International application No.
PCT/JP2020/009785

| Patent Documents referred to in the Report | Publication Date | Patent Family | Publication Date |
|---|---|---|---|
| JP 2018-115143 A | 26.07.2018 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018115143 A
- JP 10158290 A
- JP 2012201615 A
- JP 2009263279 A
- JP 2008239576 A
- JP 2005053873 A
- JP 2005023021 A
- JP 2004182687 A
- JP 2004051533 A
- JP 2008074757 A
- JP 2288890 A


### Non-patent literature cited in the description


- **MICHIHIRO HATTORI.** Science of Skin Care. Shokabo, 25 February 1997, 6-14, 15-83
- *CHEMICAL ABSTRACTS,* 93375-50-9